(19) Europäisches Patentamt
European Patent Office
Office européen des brevets

(11) EP 3 804 705 B1

(12) **EUROPEAN PATENT SPECIFICATION**

(45) Date of publication and mention of the grant of the patent:
**13.09.2023 Bulletin 2023/37**

(21) Application number: **18922061.9**

(22) Date of filing: **07.06.2018**

(51) International Patent Classification (IPC):
***A61K 31/05*** (2006.01) ***A61K 31/155*** (2006.01)
***A61K 31/64*** (2006.01) ***A61P 3/10*** (2006.01)
***A61P 3/06*** (2006.01) ***A61K 45/06*** (2006.01)

(52) Cooperative Patent Classification (CPC):
(C-Sets available)
**A61K 45/06; A61K 31/05; A61K 31/155; A61K 31/64; A61P 3/06; A61P 3/10** (Cont.)

(86) International application number:
**PCT/CN2018/090226**

(87) International publication number:
**WO 2019/232740 (12.12.2019 Gazette 2019/50)**

(54) **PHARMACEUTICAL COMPOSITION FOR PREVENTING DIABETES AND USE THEREOF**

PHARMAZEUTISCHE ZUSAMMENSETZUNG ZUR VORBEUGUNG VON DIABETES UND IHRE VERWENDUNG

COMPOSITION PHARMACEUTIQUE DESTINÉE À PRÉVENIR LE DIABÈTE ET UTILISATION CORRESPONDANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**14.04.2021 Bulletin 2021/15**

(73) Proprietor: **Hanyi Bio-Technology Company Ltd.**
**Beijing 100020 (CN)**

(72) Inventors:
- **ZHANG, Ke**
  **Beijing 100020 (CN)**
- **TAN, Xin**
  **Beijing 100020 (CN)**
- **YU, Zhaohui**
  **Beijing 100020 (CN)**
- **LI, Xiangdong**
  **Beijing 100020 (CN)**
- **LIAN, Meng**
  **Beijing 100020 (CN)**

(74) Representative: **Murgitroyd & Company**
**Murgitroyd House**
**165-169 Scotland Street**
**Glasgow G5 8PL (GB)**

(56) References cited:
**WO-A1-2005/077348**
**WO-A1-2007/138322**
**WO-A1-2016/181394**
**WO-A1-2018/064098**
**CN-A- 101 977 596**
**CN-A- 103 945 841**
**CN-A- 106 943 373**
**US-A1- 2017 165 209**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/05, A61K 2300/00;**
**A61K 31/155, A61K 2300/00;**
**A61K 31/64, A61K 2300/00**

## Description

### Field

The present invention belongs to the field of medicine, and relates to a pharmaceutical composition for preventing and treating diabetes and use thereof.

### Background

At present, the prevalence rate of diabetes is rapidly increasing, and diabetes has become another important chronic non-communicable disease that seriously endangers the health of residents after cardiovascular diseases and tumors. The disability and lethality caused by diabetes and its various acute and chronic complications have become the third largest disease threatening human health in the world, which seriously affects the quality of life of patients.

According to statistics, in 2015, about 415 million people aged 20-79 in the world suffered from diabetes (morbidity was 8.8%), and another 318 million people suffered from impaired glucose tolerance (morbidity of pre-diabetes was 6.7%). China is the largest country with diabetes patients in the world. In China, the number of patients in 2015 reached 109.6 million, and 1.3 million people died of diabetes and its complications. Furthermore, IDF predicts that, without intervention, there will be 642 million diabetes patients and 481 million people with pre-diabetes worldwide in 2040, and the number of patients in China will rise to 154 million.

Metformin (referred to as Met) or metformin hydrochloride is the first choice for clinical treatment of type 2 diabetes. Since 1957, metformin has officially entered the market as a hypoglycemic drug for the treatment of type 2 diabetes. After half a century of clinical use, more and more studies have confirmed the safety and effectiveness of metformin. Therefore, metformin has gradually become the first-line drug for diabetes treatment. Because of its low price, metformin has become one of the most widely used hypoglycemic drugs in clinic. In the *Guidelines for the Prevention and Treatment of Type 2 Diabetes in China* (2017 Edition), metformin is regarded as the drug of first choice, which highlights the important position of metformin. It is pointed out in the *Guidelines* that metformin can be used in combination with insulin secretagogues, α-glucosidase inhibitors and insulin sensitizers respectively. The structural formula of metformin is shown in a Formula A below.

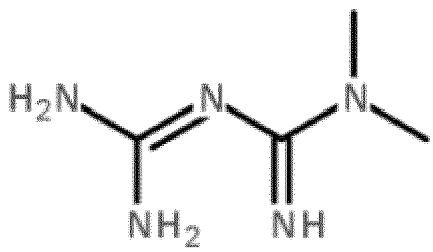

Formula A

Metformin has a variety of mechanisms of action, including delaying the uptake of glucose from the gastrointestinal tract, increasing the utilization of peripheral glucose by increasing insulin sensitivity, and inhibiting excessive liver and kidney gluconeogenesis without reducing blood glucose levels in non-diabetic patients. Clinical trials have proved that metformin can be used for a long time. The most common adverse reactions of metformin are gastrointestinal reactions, including epigastric discomfort, abdominal pain, diarrhea, nausea, vomiting, bloating, fatigue, dyspepsia, etc. In addition, 10% of diabetic patients develop lactic acidosis after taking metformin, leading some patients to give up due to their intolerance to metformin. WO2018064098A1 discloses peptides and peptide analogs and methods of treating diabetes. Glibenclamide is another common drug for treating diabetes. Glibenclamide binds specifically to a sulfonylurea receptor on a β cell membrane, and stimulates β cells to release insulin. However, long-term use of glibenclamide will cause islet atrophy; especially for patients with liver and kidney insufficiency, insulin-dependent diabetes mellitus, non-insulin-dependent diabetes mellitus with ketoacidosis, coma, severe burn, infection, trauma and leukopenia, glibenclamide is forbidden. In addition, glibenclamide can also cause the above gastrointestinal reactions similar to metformin.

More importantly, metformin and glibenclamide are also the most common dual drugs for treating type 2 diabetes. The above adverse reactions cause some patients to give up treatment with metformin and/or glibenclamide due to their intolerance.

Therefore, it is particularly urgent to find new specific therapeutic drugs to reduce the adverse reactions of metformin and/or glibenclamide while lowering blood sugar.

**Summary**

The present invention provides a pharmaceutical composition according to claim 1 to 4. The inventors have found through in-depth research that while cannabidiol can effectively inhibit type 1 and type 2 diabetes, cannabidiol can also reduce adverse reactions caused by metformin, thereby providing the following invention:
One aspect of the present invention relates to a pharmaceutical composition, including:

cannabidiol and/or a pharmaceutically acceptable salt or ester thereof; and
one or more hypoglycemic agents;
optionally, the pharmaceutical composition further includes one or more pharmaceutically acceptable auxiliary materials.

In the pharmaceutical composition, the hypoglycemic agent is selected from a group consisting of metformin, metformin hydrochloride and glibenclamide.
In one or more embodiments of the present invention, in the pharmaceutical composition, the pharmaceutical composition consists of cannabidiol and/or a pharmaceutically acceptable salt or ester thereof, one or more hypoglycemic agents and one or more pharmaceutically acceptable auxiliary materials.
In the pharmaceutical composition, a weight ratio of the cannabidiol and/or the pharmaceutically acceptable salt or ester thereof to the hypoglycemic agent is:
(10: 1) to (1:10), (5:1) to (1:10), (3:1) to (1:5), (2:1) to (1:5), (1:1) to (1:5), (1:1) to (1:3), (1:1) to (1:2), 1:1, 1:2 or 1:3. Here and hereinafter, unless otherwise specified, the weight ratio refers to a ratio of the total weight of the cannabidiol and/or the pharmaceutically acceptable salt or ester thereof to the total weight of all hypoglycemic agents.
In one or more embodiments of the present invention, the pharmaceutical composition is used for treating and/or preventing a disease or symptom selected from:

diabetes (such as type 1 diabetes or type 2 diabetes), hyperlipidemia, adverse reaction caused by metformin or adverse reaction caused by glibenclamide;
preferably, the diabetes is accompanied by at least one of the following conditions or symptoms: hepatic and renal insufficiency, ketoacidosis, coma, severe burn, infection, trauma, leukopenia, hyperlipidemia, retinopathy or chronic renal failure;
preferably, the adverse reaction caused by metformin is at least one of the following conditions or symptoms caused by taking metformin: upper abdominal discomfort, abdominal pain, diarrhea, nausea, vomiting, bloating, fatigue, indigestion or lactic acidosis;
preferably, the adverse reaction caused by glibenclamide is at least one of the following conditions or symptoms caused by taking glibenclamide: upper abdominal discomfort, abdominal pain, diarrhea, nausea, vomiting, bloating, fatigue, indigestion, lactic acidosis or islet atrophy.

The preparation form of the pharmaceutical composition can be any pharmaceutically acceptable dosage forms, including tablets, sugar-coated tablets, film-coated tablets, enteric-coated tablets, capsules(such as hard capsules, soft capsules), oral liquids, buccal tablets, granules, dissolved granules, pills, pulvis, ointments, sublimed preparations, suspensions, powders, solutions, injections, suppositories, soft ointments, hard ointments, creams, sprays, drops, and patches; preferably, the pharmaceutical composition is in oral dosage forms, such as capsules, tablets, oral liquids, granules, pills, pulvis, sublimed preparations, ointments, etc. The oral dosage forms may contain common excipients, such as binders, fillers, diluents, tableting agents, lubricants, disintegrating agents, coloring agents, flavoring agents and wetting agents, and the tablets may be coated if necessary. Suitable fillers include cellulose, mannitol, lactose and other similar fillers; suitable disintegrating agents include starch, polyvinylpyrrolidone and starch derivatives, such as sodium starch glycolate; suitable lubricants include, for example, magnesium stearate; and suitable pharmaceutically acceptable wetting agents include sodium dodecyl sulfate.
Preferably, the pharmaceutical composition is an oral preparation.
It should be pointed out that a dosage and method of using cannabidiol as an active ingredient depend on many factors, including a patient's age, weight, gender, natural health status, nutritional status, activity intensity of the compound, medication cycle, metabolic rate, severity of the disease and subjective judgment of a physician.
Some terms related to the present invention are explained below.
In the present invention, the term "Cannabidiol" (abbreviated as CBD) is one of cannabinoids, with a structural formula as shown in the following Formula I:

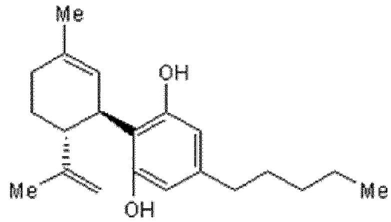

Formula I

The cannabidiol, i.e., the compound of Formula I, can be commercially available (e.g., purchased from Sigma, etc.) or synthesized by the prior art by using commercially available raw materials. After synthesis, it can be further purified by column chromatography, liquid-liquid extraction, molecular distillation or crystallization. Cannabidiol can also be extracted from industrial hemp at any time.

In some embodiments of the present invention, the pharmaceutically acceptable salt of cannabinol may be a salt formed from cannabinol (CBD) with sodium hydroxide, potassium hydroxide, calcium hydroxide, magnesium hydroxide, aluminum hydroxide, lithium hydroxide, zinc hydroxide, barium hydroxide, ammonia, methylamine, dimethylamine, diethylamine, methylpyridine, ethanolamine, diethanolamine, triethanolamine, ethylenediamine, lysine, arginine, ornithine, choline, N,N'-benzhydrylethylenediamine, chloroprocaine, procaine, N-benzylphenylethylamine, N-methylglucosamine piperazine, tris(hydroxymethyl)-aminomethane, etc.

In some embodiments of the present invention, the pharmaceutically acceptable ester of cannabidiol can be a monoester formed by cannabidiol and a $C_0$-$C_6$ alkyl carboxylic acid, or a diester formed by cannabidiol and two same or different $C_0$-$C_6$ alkyl carboxylic acids, the $C_0$-$C_6$ alkyl carboxylic acid can be linear alkyl carboxylic acid, branched alkyl carboxylic acid or cycloalkyl carboxylic acid, for example, HCOOH, $CH_3COOH$, $CH_3CH_2COOH$, $CH_3(CH_2)_2COOH$, $CH_3(CH_2)_3COOH$, $CH_3(CH_2)_4COOH$, $(CH_3)_2CHCOOH$, $(CH_3)_3CCOOH$, $(CH_3)_2CHCH_2COOH$, $(CH_3)_2CH(CH_2)_2COOH$, $(CH_3)_2CH(CH_3)CHCOOH$, $(CH_3)_3CCH_2COOH$, $CH_3CH_2(CH_3)_2CCOOH$, cyclopropane carboxylic acid, cyclobutane carboxylic acid and cyclopentane carboxylic acid.

The term "type 1 diabetes" has a meaning well known to those skilled in the art, and its original name is insulin-dependent diabetes, which mostly occurs in children and adolescents, and can also occur at various ages, mainly as autoimmune diseases, accounting for about 5% of all diabetes. The onset of the disease is sharp, insulin in the body is absolutely insufficient, and ketoacidosis is easy to occur. It is necessary to treat with insulin to obtain a satisfactory curative effect, otherwise it will endanger life.

The term "type 2 diabetes" has a meaning well known to those skilled in the art, and its original name is adult onset diabetes, which usually occurs after 35-50 years old, accounting for 90% or above of diabetic patients. The ability to produce insulin in patients with type 2 diabetes is not completely lost. Some patients even produce too much insulin, but has poor utilization effect of insulin. Therefore, insulin in patients is relatively deficient, and the utilization efficiency of insulin can be improved by oral medication. However, some patients still need insulin treatment in the later stage.

The term "effective amount" refers to a dose that can achieve treatment, prevention, alleviation and/or remission of the diseases or conditions of the present invention in a subject.

The term "subject" may refer to a patient or other animal, especially a mammal, such as a human, a dog, a monkey, a cow, a horse, etc., which receives the composition of the present invention to treat, prevent, alleviate and/or ameliorate the diseases or conditions described in the present invention.

The term "disease and/or condition" refers to a physical state of the subject, which is related to the disease and/or condition of the present invention.

In the present invention, unless otherwise specified, the first pharmaceutical preparation and the second pharmaceutical preparation are only for clarity of reference, and do not have sequential meanings.

Advantageous effects of the present invention:
The present invention achieves one or more of the following technical effects:

(1) CBD can significantly inhibit type 1 diabetes and type 2 diabetes in mammals (such as mice or humans) and significantly increase insulin sensitivity; and has a better effect than that of metformin alone;
(2) CBD combined with metformin can significantly reduce hyperlipidemia and high cholesterol induced by STZ and high-fat diet, and can increase insulin secretion;
(3) CBD can significantly inhibit diarrhea and lactic acidemia caused by metformin; and
(4) CBD can also significantly reduce the urinary total protein in mice with adriamycin-induced renal failure.

## Brief Description of the Drawings

FIG. 1A: fasting blood glucose results of mice in each group.
FIG. 1B: results of glucose tolerance test (GTT) in mice.
FIG. 1C: results of insulin tolerance test (ITT) in mice.
FIG. 2A: fasting blood glucose of mice.
FIG. 2B: results of glucose tolerance test (GTT) in mice.
FIG. 2C: results of insulin tolerance test (ITT) in mice.
FIG. 3: relative blood glucose level of DB/DB mice after administration treatment.
FIG. 4A: results of diarrhea index of mice on the first day of induction.
FIG. 4B: results of diarrhea index of mice on the second day of induction.
FIG. 4C: results of diarrhea index of mice on the third day of induction.
FIG. 4D: results of diarrhea index of mice on the fourth day of induction.
FIG. 4E: results of diarrhea index of mice on the fifth day of induction.
FIG. 4F: results of diarrhea index of mice on the sixth day of induction.
FIG. 4G: results of diarrhea index of mice on the seventh day of induction.
FIG. 5: lactic acid concentration in mouse blood.
FIG. 6A: cholesterol insulin level in mouse serum.
FIG. 6B: triglyceride level in mouse serum.
FIG. 6C: high-density cholesterol level in mouse serum.
FIG. 6D: low-density cholesterol level in mouse serum.
FIG. 6E: insulin level in mouse serum.
FIG. 7: total protein level of urine reduced by CBD.
FIG. 8A: fasting blood glucose of 3 diabetic volunteers after CBD treatment.
FIG. 8B: glycosylated hemoglobin level of 3 diabetic volunteers after CBD treatment.

## Detailed Description of the Embodiments

Embodiments of the present invention will be described in detail with reference to examples, but those skilled in the art will understand that the following examples are only used to illustrate the present invention and should not be regarded as limiting the scope of the present invention. If the specific conditions are not indicated in the embodiment, the conventional conditions or the conditions suggested by the manufacturer shall be followed. The reagents or instruments used are conventional products that are commercially available if not indicating the manufacturers.

CBD, provided by Hanyi Biotechnology (Beijing) Co., Ltd. China;
Metformin, purchased from Aladdin (the structure is the same as the previous formula A);
Insulin, purchased from China Agricultural University hospital;
Streptozotocin (STZ), purchased from Aladdin.
C57 male mice, purchased from Charles River;
ICR male mice, purchased from Charles River;
ICR female mice, purchased from Charles River;
Kunming white male mice, purchased from Charles River.

<u>Example 1: Establishment of mouse model of type 1 diabetes and drug treatment</u>

1) Conventional induction method of type 1 diabetes model (multiple low-dose induction method)

C57 male mice aged 4-6 weeks were used as experimental animals. After the mice were fasted for 10-12 hours, they were injected with 50 mg/kg of streptozotocin (STZ) intraperitoneally for 5 consecutive days. Fasting blood glucose levels of mice on the 6th and 10th days were measured to determine whether the induction was successful (the induction was considered successful when fasting blood glucose was greater than 11.1 mmol/L). The mice were applied to Group 2 to 6 in the following step 2).

2) Experimental grouping and administration

The mice were randomly divided into 6 groups with 10 mice in each group:

Group 1: blank control group, fed with normal feed and intraperitoneally injected with a buffer solution at a dose of 100 μl/mouse, once a day for 5 consecutive days;

wherein, the buffer solution was a citric acid buffer solution with a concentration of 0.1 mol/L and pH of 4.2 and was sterilized by suction filtration.

Group 2: diabetes model group, fed with normal feed and intraperitoneally injected with streptozotocin;

Group 3: CBD prevention group, fed with normal feed, intraperitoneally injected with streptozotocin, and given CBD by gavage from the first day of induction, 50 mg/kg, once a day;

Group 4: CBD treatment group, fed with normal feed, intraperitoneally injected with streptozotocin, and given CBD by gavage after successful induction, 50 mg/kg, once a day;

Group 5: positive drug control group, fed with normal feed, intraperitoneally injected with streptozotocin, and injected with insulin after successful induction, 0.2 U/kg, once a day; and

Group 6: metformin group, fed with normal feed, intraperitoneally injected with streptozotocin, and given metformin by gavage after successful induction, 50 mg/kg, once a day.

3) Glucose tolerance test (GTT) and insulin tolerance test (ITT)

Fasting blood glucose of mice in each group was detected every other three to four weeks; after one month of administration (the model was stable), a glucose tolerance test (GTT) was carried out. The mice were fasted for 10 hours, and was injected with 20% (w/w) glucose intraperitoneally according to their body weights (10μl/g). The blood glucose of the mice before injection (0 min) and at 15 min, 30 min, 60 min and 120 min after injection were measured respectively.

One week after GTT, an insulin tolerance test (ITT) was carried out. The mice were fasted for 10 hours, and injected with 0.75 U/kg of insulin intraperitoneally. The blood glucose of the mice before injection (0 min) and at 15 min, 30 min, 60 min and 120 min after injection were measured respectively.

4) Experimental results

The results are as shown in FIG. 1A, FIG. 1B and FIG. 1C respectively.

It can be seen from FIG. 1A that STZ can induce a mouse diabetes model; both the CBD prevention group and the CBD treatment group could significantly reduce the blood glucose increase caused by STZ after 10 days. In addition, the CBD prevention group had a better effect than the CBD treatment group.

It can be seen from the GTT results in FIG. 1B that CBD could significantly enhance regulation of glucose in the mice. In addition, the CBD prevention group had a better effect than the CBD treatment group.

It can be seen from the ITT results in FIG. 1C that CBD could significantly enhance sensitivity of the mice to insulin, regulate their blood glucose and alleviate the symptoms of type 1 diabetes. In addition, the CBD prevention group had a better effect than the CBD treatment group.

In addition, metformin should have no effect on type 1 diabetes, and it is not be used as a clinical drug for treating type 1 diabetes.

<u>Example 2: Establishment of mouse model of type 2 diabetes and drug treatment</u>

1) Induction method of type 2 diabetes model (multiple low-dose induction and high-fat diet induction)

C57 male mice aged 4-6 weeks were used as experimental animals. The mice were fed with high-fat feed. After the mice were fasted for 10-12 hours, they were injected with 50 mg/kg of streptozotocin (STZ) intraperitoneally for 5 consecutive days. Fasting blood glucose levels of the mice on the 6th and 10th days were measured to determine whether the induction was successful (the induction was considered successful when the fasting blood glucose was more than 11.1 mmol/L, and it was successfully on the 32nd day in the experiment). On the 6th day, the mice whose blood glucose did not reach the standard (which were not induced successfully) were injected with streptozotocin intraperitoneally at a dose of 80 mg/kg on the 8th day. The mice were applied to Group 2 to 8 in the following step 2).

High-fat feed was formed by adding 60% fat to normal feed.

2) Experimental grouping and administration

The mice were randomly divided into 8 groups with 10 mice in each group:

Group 1: blank control group, fed with high-fat feed and injected with a buffer solution intraperitoneally at a dose of 100 μl/mouse, once a day for 5 consecutive days;

wherein, the buffer solution was a citric acid buffer solution with a concentration of 0.1 mol/L and pH of 4.2 and was sterilized by suction filtration.

Group 2: diabetes model group, fed with high-fat feed and injected intraperitoneally with streptozotocin;

Group 3: CBD prevention group, fed with high-fat feed, intraperitoneally injected with streptozotocin, and given CBD by gavage from the first day of induction, 50 mg/kg, once a day;

Group 4: CBD treatment group, fed with high-fat feed, intraperitoneally injected with streptozotocin, and given CBD by gavage every day (50 mg/kg) after successful induction (the 32nd day);

Group 5: metformin group, fed with high-fat feed, intraperitoneally injected with streptozotocin, and given metformin from the 32nd day, 50 mg/kg, once a day;

Group 6: metformin + CBD group, fed with high-fat feed, intraperitoneally injected with streptozotocin, and administrated (metformin 50 mg/kg + CBD 50 mg/kg) by gavage every day from the 32nd day;

Group 7: metformin + glibenclamide (Gli) group, fed with high-fat feed, intraperitoneally injected with streptozotocin; and administrated drugs (metformin 50 mg/kg + Gli 0.758 mg/kg) by gavage every day after successful induction; and

Group 8: glibenclamide + CBD group, fed with high-fat feed, intraperitoneally injected with streptozotocin, and administrated (Gli 0.758 mg/kg + CBD 50 mg/kg) by gavage every day after successful induction.

3) Glucose tolerance test (GTT) and insulin tolerance test (ITT)

One month after administration (after the type 2 diabetes model was stabilized,), a glucose tolerance test (GTT) was carried out. The mice were fasted for 10 hours, and injected with 20% (w/w) glucose was intraperitoneally according to their body weights (10 μl/g). The blood glucose of the mice before injection (0 min) and at 15 min, 30 min, 60 min and 120 min after injection were measured respectively.

One month after administration (after the model was stabilized), an insulin tolerance test (ITT) was carried out. The mice were fasted for 10 hours, and injected with 0.75 U/kg of insulin intraperitoneally. The blood glucose of mice before injection (0 min) and at 15 min, 30 min, 60 min and 120 min after injection were measured respectively.

The experimental results are shown in FIG. 2A, FIG. 2B and FIG. 2C respectively.

It can be seen from the results of FIG. 2A that STZ and high-fat feed could cause blood glucose elevation of the mice, while CBD could significantly reduce the blood glucose elevation induced by STZ and high-fat feed.

It can be seen from the results of glucose tolerance test in mice in FIG. 2B that CBD could improve regulation of glucose in the mice induced by STZ and high-fat feed, and control blood glucose better.

It can be seen from the results of insulin tolerance test in mice in FIG. 2C that CBD could improve sensitivity of the mice to insulin and better regulate their blood glucose, and alleviate the symptoms of type 2 diabetes.

It can be seen from FIG. 2A to FIG. 2C that the blood glucose of the mice in metformin + CBD group (Group 6) was obviously lower than that of other groups, and both the glucose tolerance and insulin sensitivity of the mice in this group were improved obviously.

In addition, FIG. 2A to FIG. 2C show that the CBD prevention group had a better effect than the CBD treatment group.

Example 3: Study on the effect of combined use of CBD and metformin on type 2 diabetes

In order to study the effect of combined use of CBD and metformin, adult DB/DB mice (spontaneous type 2 diabetic mice, purchased from Model Animal Center of Nanjing University) were randomly divided into 5 groups, with 6 mice in each group:

Group 1: control group, no treatment;

Group 2: CBD alone administration group (50 mg/kg);

Group 3: low-dose metformin group (100 mg/kg, calculated by referring to the lowest dose for human, and the dose for mice =9.1 × the dose for human);

Group 4: low-dose metformin (100 mg/kg) + CBD (50 mg/kg) group; and

Group 5: high-dose metformin group (250 mg/kg, calculated by referring to the lowest dose for human, and the dose for mice =9.1 × the dose for human).

Group 3 and Group 5 were both positive control groups of the treatment group, Group 3 was low-dose metformin group, and Group 5 was high-dose metformin group. Clinically, patients receive low-dose metformin treatment at first, and then the dose of metformin is gradually increased according to changes in blood glucose, but it cannot exceed the maximum dose. The experiment of this example is to prove that the effect of combined use of CBD and metformin is better than low-dose metformin and high-dose metformin.

The administration routes of the above five groups were all intragastric administration, once a day.

After 4 weeks of administration, blood glucose of mice in each group was measured. The relative blood glucose results of mice in each group are shown in FIG. 3.

The results showed that during the administration period, the blood glucose of mice in the CBD + metformin group (Group 4) decreased the most sharply fastest and obviously. After 5 weeks of administration, the fasting blood glucose of mice treated with CBD and metformin was significantly lower than those treated with metformin alone (Groups 3 and 5), and also lower than that treated with CBD alone (Group 2).

The results showed that CBD and the combination of CBD and metformin could reduce blood glucose in DB/DB mice, and the combination of CBD and metformin had a better effect.

Example 4: Establishment of mouse diarrhea model and drug treatment

1) Induction method of mouse diarrhea model (induction method of mouse diarrhea by *Folium Sennae)*

ICR female mice of 4-6 weeks old were used as experimental animals. The mice ate and drank normally, and were given 8% (w/w) *Folium Sennae* solution by gavage, 0.2 ml/mouse. Diarrhea index of the mice was counted every other one and a half hours for 6 hours. *Folium Sennae* was purchased from Tongrentang Pharmacy in Beijing. The 8% (w/w) *Folium Sennae* solution was prepared by boiling 8 parts by weight of *Folium Sennae* and 92 parts by weight of water in a conical flask for 10min.

2) Experimental grouping and administration

The mice were randomly divided into 7 groups with 10 mice in each group:

Group 1: the mice were given the same volume of water by gavage every day;
Group 2: the mice were given 150 mg/kg of metformin by gavage every day;
Group 3: the mice were given 50 mg/kg of CBD by gavage every day;
Group 4: the mice were given 8% *Folium Sennae* solution by gavage every day;
Group 5: the mice were given a mixed solution of 8% *Folium Sennae* solution and 150 mg/kg of metformin by gavage every day;
Group 6: the mice were given a mixed solution of 8% *Folium Sennae* solution and 50 mg/kg of CBD by gavage every day; and
Group 7: the mice were given a mixed solution of 8% *Folium Sennae* solution, 150 mg/kg of metformin and 50 mg/kg of CBD by gavage every day.

3) Detection and statistical method

Every day after gavage, the diarrhea index of each mouse was counted every other one and a half hours, 4 times in total (once every 6 hours).

$$\text{Diarrhea index} = \text{loose stool rate} * \text{loose stool grade}$$

Wherein,
Diarrhea rate: the number of animals with loose stools in this group/the total number of animals in this group * 100%;
Loose stool rate: the number of loose stools per animal/the total number of stools; and
Loose stool grade: the hardness of loose stool graded according to the size of stains on filter paper with loose stool, as shown in Table 1 below.

Table 1: Loose stool grade

| Grade | 0 | 1 | 2 | 3 | 4 |
|---|---|---|---|---|---|
| Diameter of stains (cm) | No stains | <1 | 1-1.9 | 2-2.9 | >3 |

4) Experimental results

The results are as shown in FIG. 4A to FIG. 4G respectively.

FIG. 4A to FIG. 4G show the results of the diarrhea index of mice from the first day to the seventh day after

induction. It can be seen from the statistical results of the diarrhea index of mice in each group in FIG. 4A to FIG. 4G that *Folium Sennae* could cause diarrhea in mice, metformin could aggravate diarrhea induced by *Folium Sennae* in mice, and CBD could significantly alleviate diarrhea induced by *Folium Sennae* in mice and diarrhea induced and aggravated by metformin.

Example 5: Establishment of mouse lactic acidosis model and drug treatment

1) Induction method of mouse lactic acidosis model
ICR male mice aged 4-6 weeks were used as experimental animals. The mice were administrated by gavage with metformin (260 mg/kg) for induction for three times, with an interval of 12 hours.
2) Experimental grouping and administration
The mice were randomly divided into seven groups with five mice in each group:

Group 1: control group, no treatment;
Group 2: given water by gavage every day;
Group 3: given CBD by gavage once a day, 50 mg/kg;
Group 4: given 260 mg/kg of metformin by gavage in the morning and evening on the first day, and 260 mg/kg of metformin by gavage in the next morning, and sampled at 4 pm in the next day;
Group 5: given 260 mg/kg of metformin by gavage in the morning and evening on the first day, and 260 mg/kg of metformin by gavage in the next morning, given 50 mg/kg of CBD at the same time metformin was given by gavage, and sampled at 4 pm in the next day.
Group 6: injected with 260 mg/kg of metformin by caudal vein every other one hour for four times, and sampled one hour after the fourth injection; and
Group 7: given 50 mg/kg of CBD by gavage half an hour before caudal vein injection, injected with 260 mg/kg of metformin by caudal vein half an hour later, once every other hour, four times in total, and sampled one hour after the fourth injection.

3) Experimental design: blood samples were collected to detect lactic acid concentration before treatment, and blood samples of mice in each group were collected to detect lactic acid concentration after treatment.
4) The experimental results are shown in FIG. 5.

The results show that metformin could induce lactic acidemia in mice, while CBD could significantly inhibit the increase of lactic acid in blood induced by metformin.

Example 6: Study on the effect of combined use of CBD and metformin on reducing blood lipid level in mice induced by high-fat feed

1) Induction method of diabetes model (multiple low-dose induction method)
Kunming white male mice aged 4-6 weeks were used as experimental animals. After the mice were fasted for 10-12 hours, they were injected with 50 mg/kg of streptozotocin (STZ) intraperitoneally for 5 consecutive days. Fasting blood glucose levels of the mice on the 6th and 10th days were measured to determine whether the induction was successful (the induction was considered successful when fasting blood glucose was greater than 11.1 mmol/L). The mice were applied to the Group 2 to 5 in the following step 3).
2) High-fat feed induction method
During the experiment, mice were fed with high-fat feed containing 60% fat on the basis of normal feed. The mice in Groups 2 to 5 were fed with high-fat feed.
3) Experimental grouping and administration
The mice were randomly divided into 5 groups with 10 mice in each group:

Group 1: blank control group, fed with normal feed and intraperitoneally injected with a buffer solution at a dose of 100 μl/mouse, once a day for 5 consecutive days;
wherein, the buffer solution was a citric acid buffer solution with a concentration of 0.1 mol/L and pH of 4.2 and was sterilized by suction filtration;
Group 2: diabetes model group, fed with high-fat feed and intraperitoneally injected with streptozotocin;

Group 3: low-dose metformin group, fed with high-fat feed, intraperitoneally injected with streptozotocin, and given low-dose metformin by gavage from the first day of induction, 100 mg/kg, once a day;
Group 4: low-dose metformin + CBD group, given low-dose metformin, 100 mg/kg, once a day; further given CBD by gavage, 50mg/kg, once a day, fed with high-fat feed, injected with streptozotocin intraperitoneally, and given low-dose metformin and CBD by gavage from the first day of induction;
Group 5: high-dose metformin group, 250 mg/kg, once a day, fed with high-fat feed, injected with streptozotocin intraperitoneally, and given high-dose metformin by gavage from the first day of induction. Group 5 served as a positive control treatment group.

4) Detection method
After two months of drug treatment, the mice were sacrificed, blood was collected from the heart, serum was taken, and levels of cholesterol, triglyceride, high-density cholesterol, low-density cholesterol and insulin in the serum were measured respectively.
5) Experimental results
The experimental results are shown in FIG. 6A to FIG. 6E, respectively.

It can be seen from FIG. 6A to FIG. 6E that combination of CBD and metformin could significantly reduce hyperlipidemia and hypercholesterolemia induced by STZ and high-fat diet, and can increase insulin secretion. In addition, FIG. 6E shows that metformin alone could not increase insulin secretion.

Example 7: CBD significantly reduces urinary total protein level in mice with chronic renal failure induced by adriamycin

A mouse model of chronic renal failure induced by adriamycin was constructed according to a conventional method of inducing kidney injury. After CBD treatment for 14 days, urinary total protein level was measured. The specific method is as follows:

1) Induction method of diabetic nephropathy model (multiple low-dose induction method) Kunming white male mice aged 4-6 weeks were used as experimental animals. After the mice were fasted for 10-12 hours, they were injected with 50 mg/kg of streptozotocin (STZ) intraperitoneally for 5 consecutive days. Fasting blood glucose levels of the mice on the 6th and 10th days were measured to determine whether the induction was successful (diabetes was considered successfully induced when fasting blood glucose was greater than 11.1 mmol/L). On the 7th day after diabetes induction, the mice in the nephropathy group were injected with adriamycin at 10 mg/kg by caudal vein. The mice were applied to the Group 3 to 6 in the following step 2).
2) Experimental grouping and administration
The experimental animals were randomly divided into 6 groups with 10 animals in each group:

Group 1: control group, fed with normal saline;
Group 2: diabetes group;
Group 3: diabetes + CBD group; given CBD by gavage, 50 mg/kg, once a day;
Group 4: diabetes + adriamycin group;
Group 5: diabetes + adriamycin + CBD group; given CBD by gavage, 50 mg/kg, once a day; and
Group 6: diabetes + adriamycin + metformin group; given metformin by gavage, 50 mg/kg, once a day.

The above Group 2 and Group 3 were set up to measure urinary total protein content when only diabetes was induced instead of chronic renal failure.
3) Detection method
After successfully inducing the mouse diabetes model, the mice were injected with adriamycin through caudal vein to induce nephropathy model. Urine was collected from the mice on the 14th day of the induction of nephropathy model, and urinary total protein was detected.
4) Experimental results
The experimental results are shown in FIG. 7.

The results show that CBD could significantly reduce urinary total protein level of mice with chronic renal failure induced by adriamycin. Nephropathy is one of the complications of diabetes, so CBD can treat chronic renal failure caused by diabetes.

Example 8: CBD significantly reduces fasting blood glucose and glycosylated hemoglobin levels in patients with type 2 diabetes

Three diabetic volunteers in clinic participated in this study: two males, weighing about 65 kg and 70 kg and aged 47 and 52 years respectively; a female, weighing about 55 kg and aged 74 years.

Every patient received oral CBD treatment at 300 mg/day for 8 weeks. Fasting blood glucose was measured at the $0^{th}$, $4^{th}$ and $8^{th}$ weeks. Glycosylated hemoglobin value was measured at the $0^{th}$ and $8^{th}$ weeks. The results are shown in FIG. 8A and FIG. 8B, respectively.

FIG. 8A shows that after 8 weeks of CBD treatment, fasting blood glucose of all three patients decreased significantly (although not to the normal range); and

FIG. 8B shows that after 8 weeks of CBD treatment, glycosylated hemoglobin of the three patients improved significantly (although not to the normal range, i.e., 4%-6%).

## Claims

1. A pharmaceutical composition, comprising:

   cannabidiol and/or a pharmaceutically acceptable salt or ester thereof; and
   one or more hypoglycemic agents;
   wherein the hypoglycemic agent is selected from a group consisting of: metformin, metformin hydrochloride and glibenclamide; and
   a weight ratio of the cannabidiol and/or the pharmaceutically acceptable salt or ester thereof to the hypoglycemic agent is (10:1) to (1:10).

2. The pharmaceutical composition according to claim 1, wherein, the pharmaceutical composition consists of cannabidiol and/or a pharmaceutically acceptable salt or ester thereof, one or more hypoglycemic agents and one or more pharmaceutically acceptable auxiliary materials.

3. The pharmaceutical composition according to any one of claims 1 to 2, wherein, a weight ratio of the cannabidiol and/or the pharmaceutically acceptable salt or ester thereof to the hypoglycemic agent is:
   (5:1) to (1:10), (3:1) to (1:5), (2:1) to (1:5), (1:1) to (1:5), (1:1) to (1:3), (1:1) to (1:2), 1:1, 1:2 or 1:3.

4. The pharmaceutical composition according to any one of claims 1 to 3 for use in the treatment and/or prevention of a disease or symptom selected from:
   diabetes, hyperlipidemia, adverse reaction caused by metformin or adverse reaction caused by glibenclamide.

## Patentansprüche

1. Eine pharmazeutische Zusammensetzung, die Folgendes beinhaltet:

   Cannabidiol und/oder ein pharmazeutisch akzeptables Salz oder einen pharmazeutisch akzeptablen Ester davon; und
   ein oder mehrere Hypoglykämika;
   wobei das Hypoglykämikum aus einer Gruppe ausgewählt ist, die aus Folgendem besteht: Metformin, Metforminhydrochlorid und Glibenclamid; und
   ein Gewichtsverhältnis des Cannabidiols und/oder des pharmazeutisch akzeptablen Salzes oder Esters davon zu dem Hypoglykämikum (10 : 1) bis (1 : 10) beträgt.

2. Pharmazeutische Zusammensetzung gemäß Anspruch 1, wobei die pharmazeutische Zusammensetzung aus Cannabidiol und/oder einem pharmazeutisch akzeptablen Salz oder Ester davon, einem oder mehreren Hypoglykämika und einem oder mehreren pharmazeutisch akzeptablen Hilfsstoffen besteht.

3. Pharmazeutische Zusammensetzung gemäß einem der Ansprüche 1 bis 2, wobei ein Gewichtsverhältnis des Cannabidiols und/oder des pharmazeutisch akzeptablen Salzes oder Esters davon zu dem Hypoglykämikum Folgendes beträgt: (5 : 1) bis (1 : 10), (3 : 1) bis (1 : 5), (2 : 1) bis (1 : 5), (1 : 1) bis (1 : 5), (1 : 1) bis (1 : 3), (1 : 1) bis (1 : 2), 1 : 1, 1 : 2 oder 1 : 3.

**4.** Pharmazeutische Zusammensetzung gemäß einem der Ansprüche 1 bis 3 zur Verwendung bei der Behandlung und/oder Vorbeugung einer Krankheit oder eines Symptoms, ausgewählt aus:
Diabetes, Hyperlipidämie, einer durch Metformin verursachten Nebenwirkung oder einer durch Glibenclamid verursachten Nebenwirkung.

## Revendications

**1.** Une composition pharmaceutique, comprenant :

du cannabidiol et/ou un sel ou ester pharmaceutiquement acceptables de celui-ci ; et un ou plusieurs agents hypoglycémiants ;
dans laquelle l'agent hypoglycémiant est sélectionné dans un groupe constitué de : la metformine, le chlorhydrate de metformine et le glibenclamide ; et
un rapport en poids du cannabidiol et/ou du sel ou ester pharmaceutiquement acceptables de celui-ci à l'agent hypoglycémiant est de (10:1) à (1:10).

**2.** La composition pharmaceutique selon la revendication 1, la composition pharmaceutique étant constituée de cannabidiol et/ou d'un sel ou ester pharmaceutiquement acceptables de celui-ci, d'un ou plusieurs agents hypoglycémiants et d'une ou plusieurs matières auxiliaires pharmaceutiquement acceptables.

**3.** La composition pharmaceutique selon l'une quelconque des revendications 1 à 2, dans laquelle un rapport en poids du cannabidiol et/ou du sel ou ester pharmaceutiquement acceptables de celui-ci à l'agent hypoglycémiant est de : (5:1) à (1:10), (3:1) à (1:5), (2:1) à (1:5), (1:1) à (1:5), (1:1) à (1:3), (1:1) à (1:2), 1:1, 1:2 ou 1:3.

**4.** La composition pharmaceutique selon l'une quelconque des revendications 1 à 3 pour utilisation dans le traitement et/ou la prévention d'une maladie ou d'un symptôme sélectionnés parmi :
le diabète, l'hyperlipidémie, un effet indésirable causé par la metformine ou un effet indésirable causé par le glibenclamide.

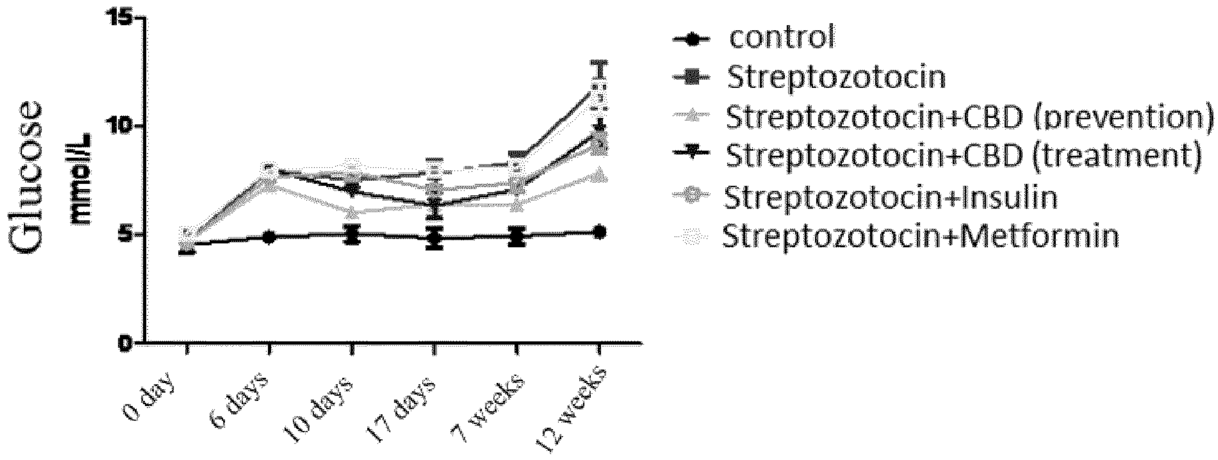


FIG. 1A

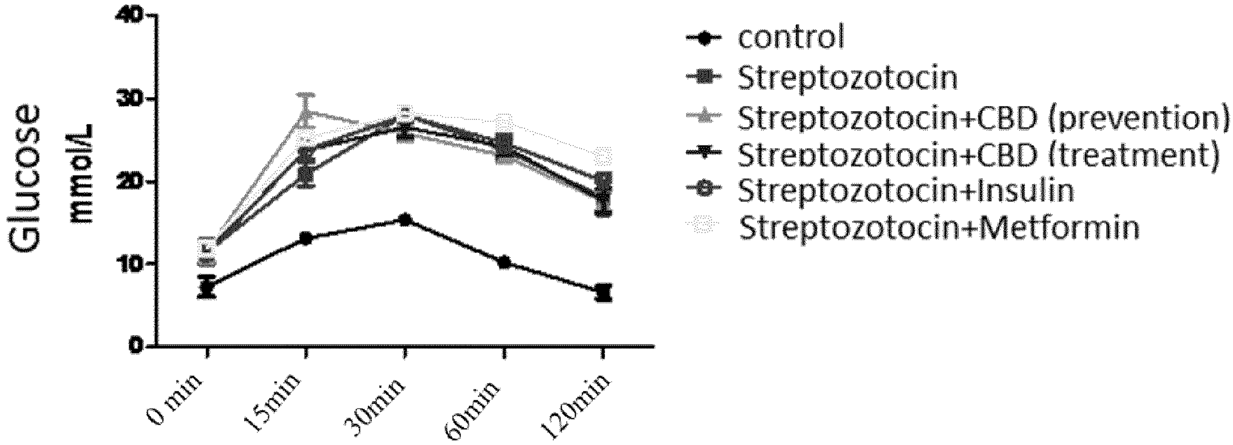


FIG. 1B

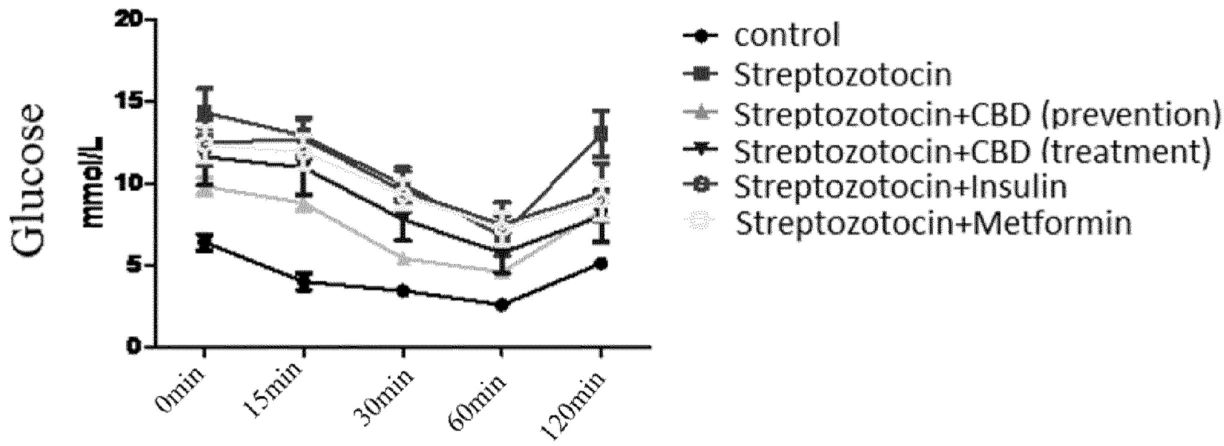


FIG. 1C

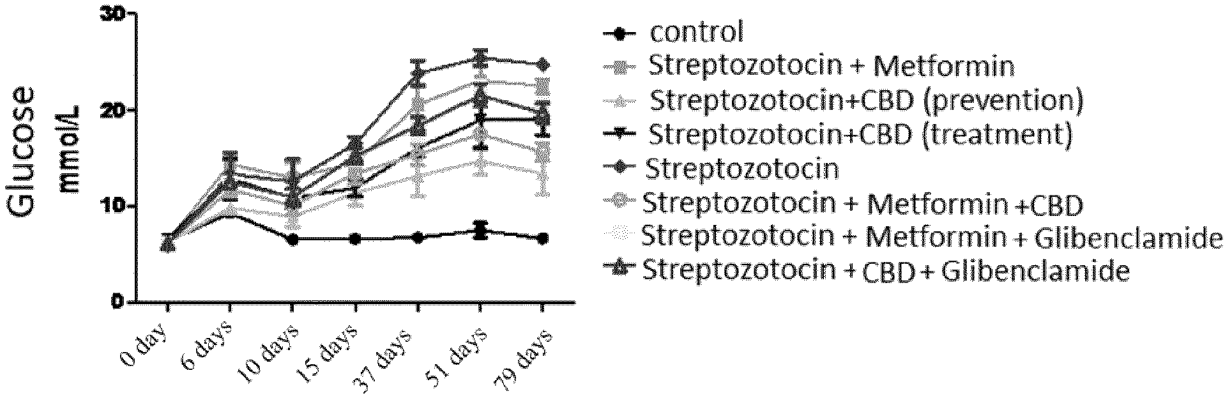


FIG. 2A

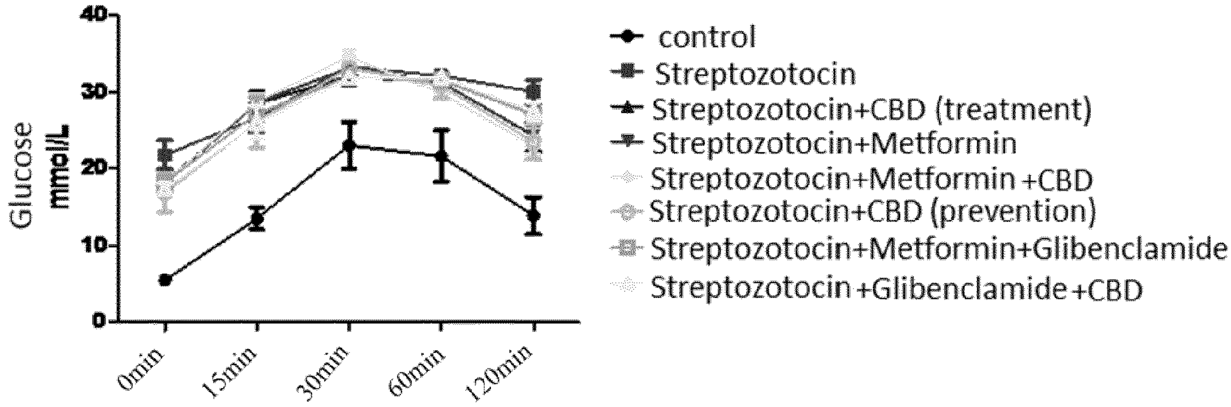


FIG. 2B

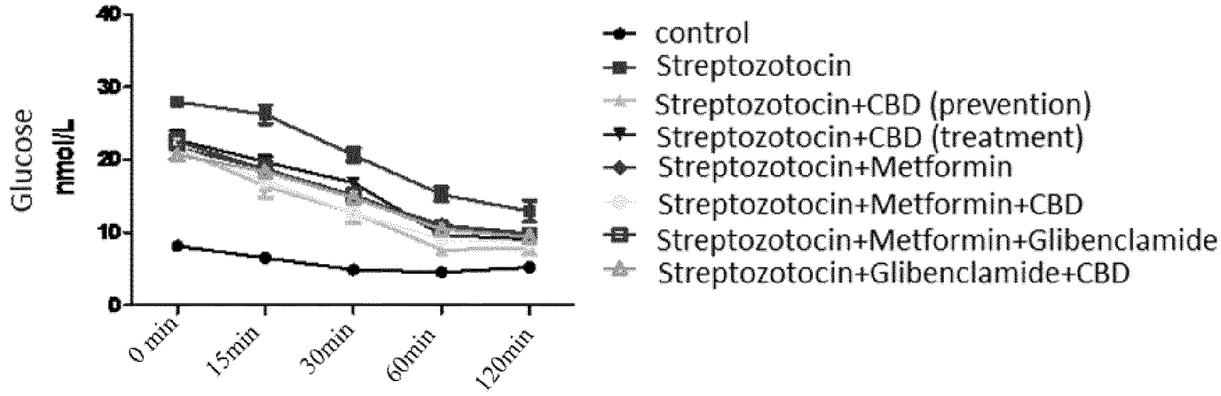


FIG. 2C

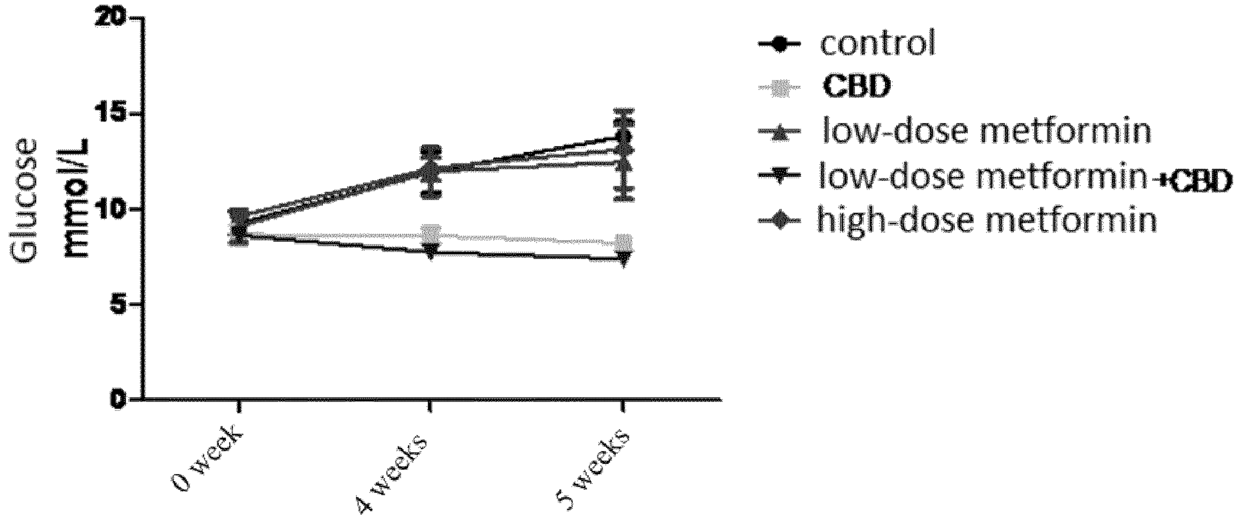


FIG. 3

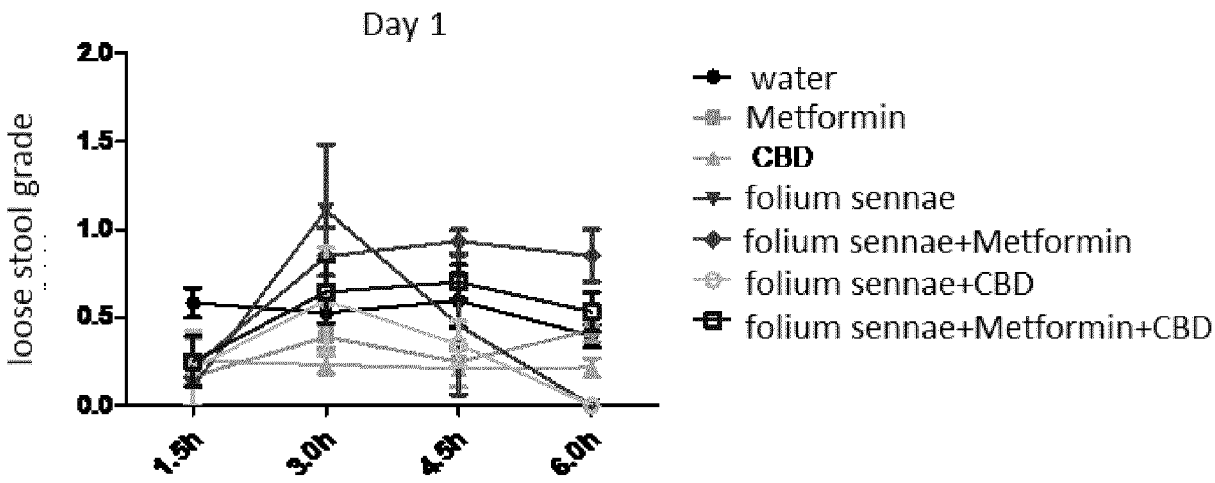


FIG. 4A

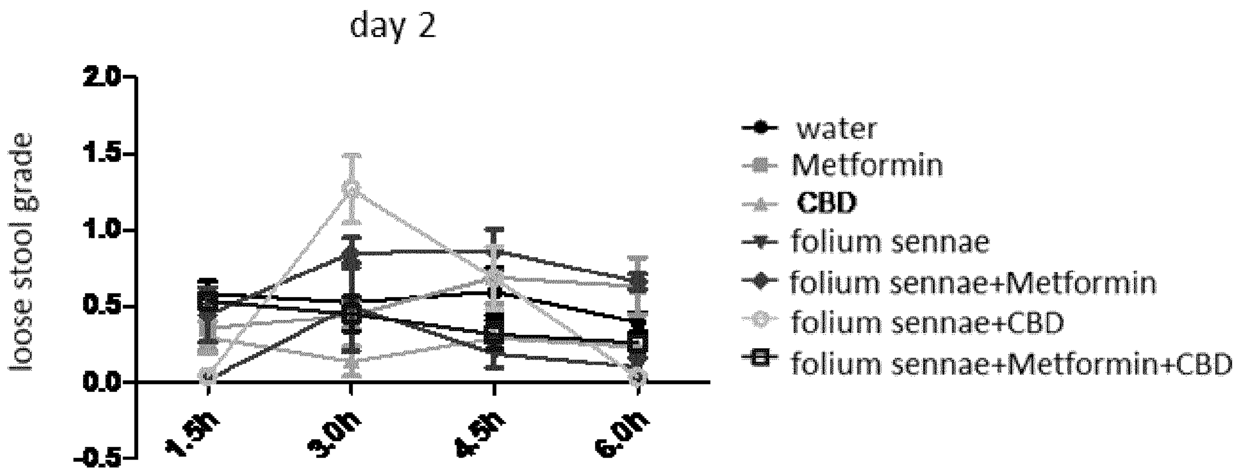


FIG. 4B

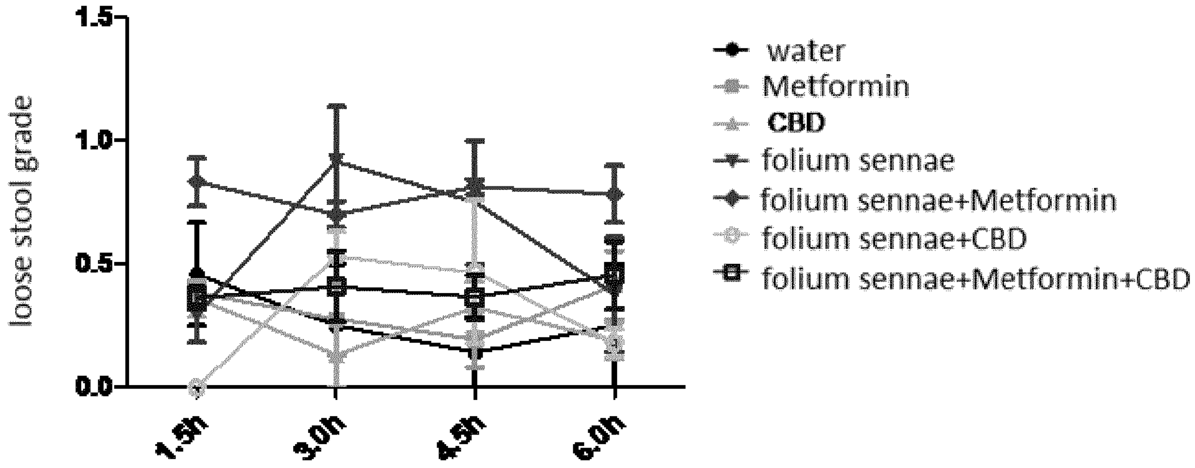


FIG. 4C

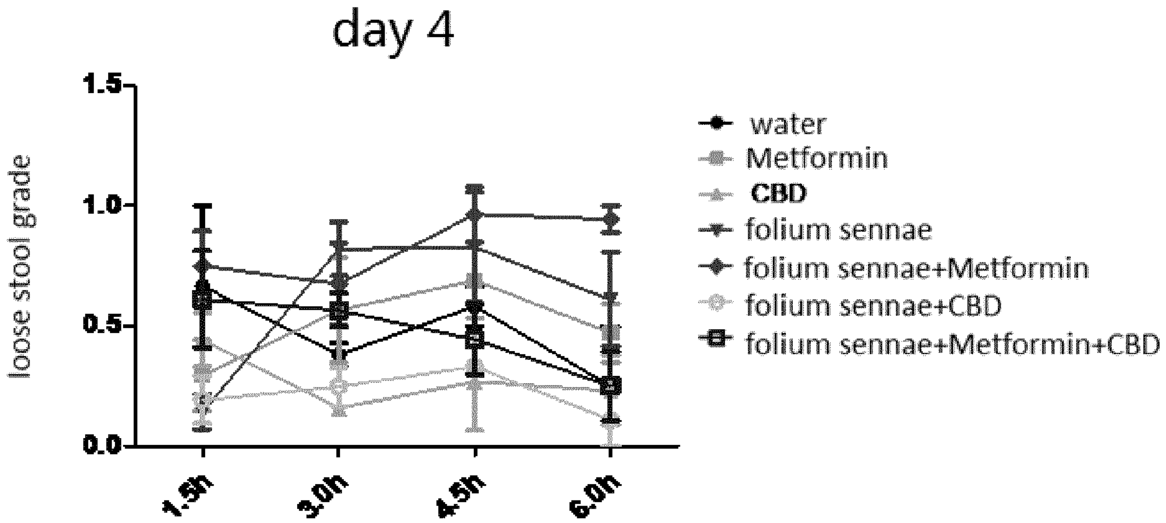


FIG. 4D

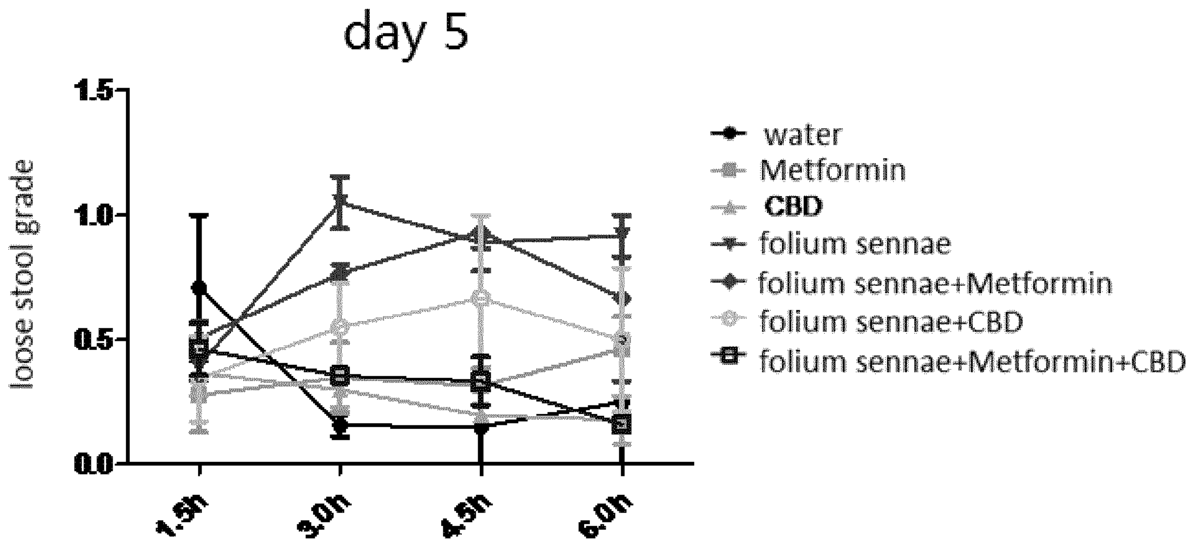


FIG. 4E

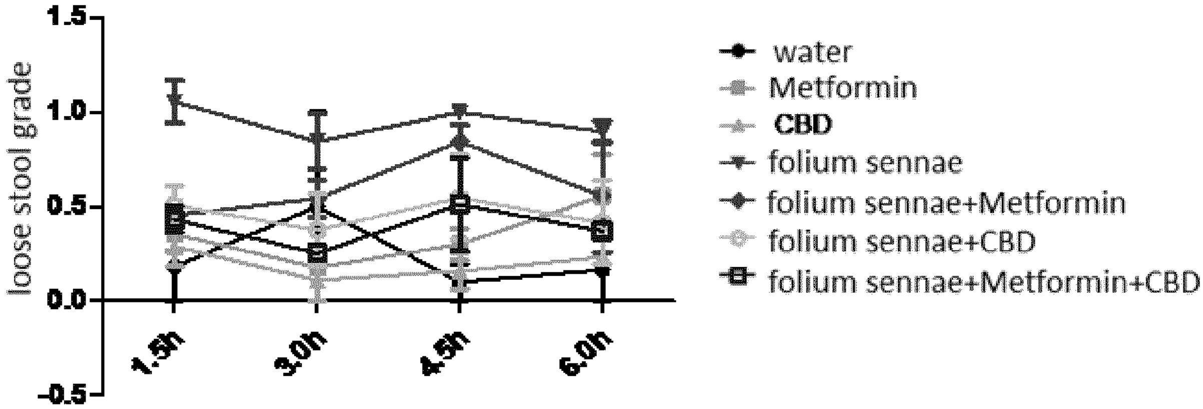
1.5
1.0
0.5
0.0
-0.5
loose stool grade
1.5h
3.0h
4.5h
6.0h
water
Metformin
CBD
folium sennae
folium sennae+Metformin
folium sennae+CBD
folium sennae+Metformin+CBD

FIG. 4F

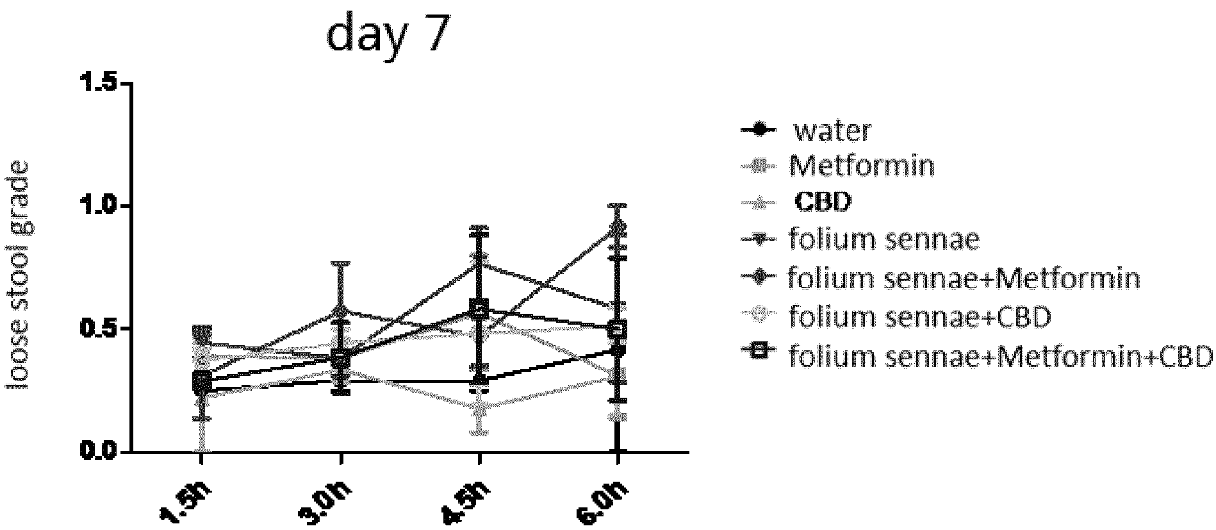
day 7
1.5
1.0
0.5
0.0
loose stool grade
1.5h
3.0h
4.5h
6.0h
water
Metformin
CBD
folium sennae
folium sennae+Metformin
folium sennae+CBD
folium sennae+Metformin+CBD

FIG. 4G

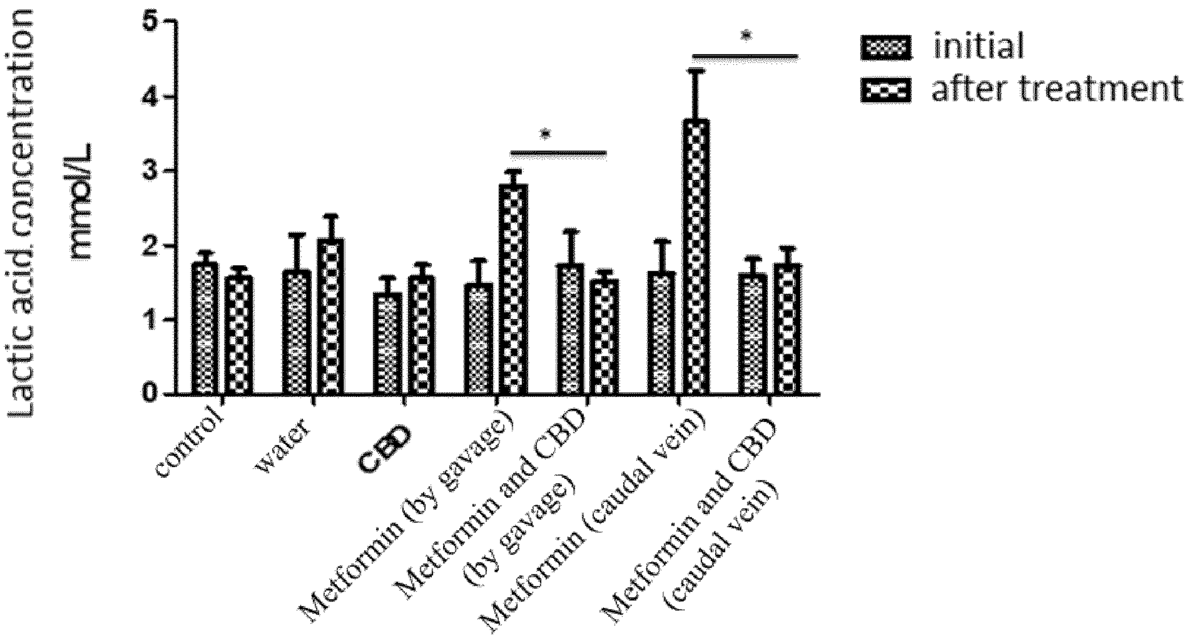


FIG. 5

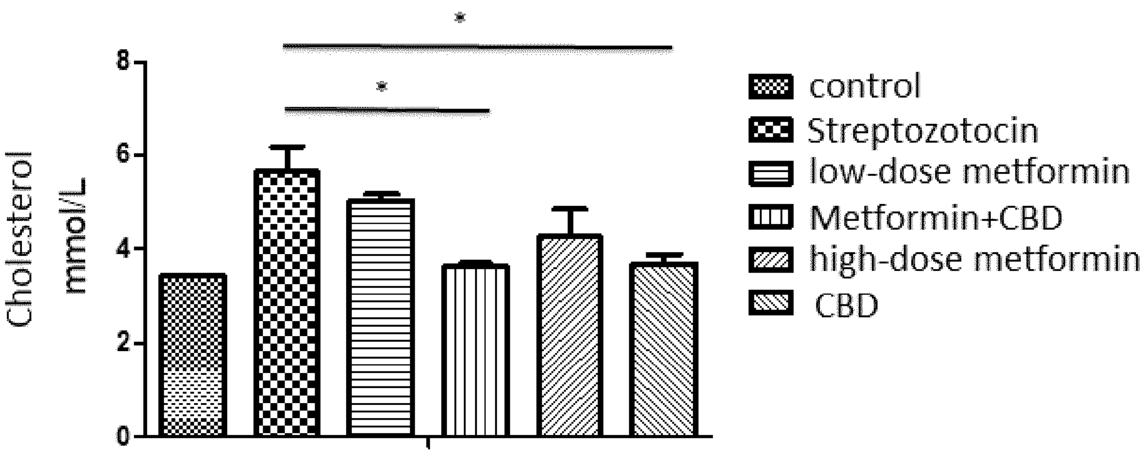


FIG. 6A

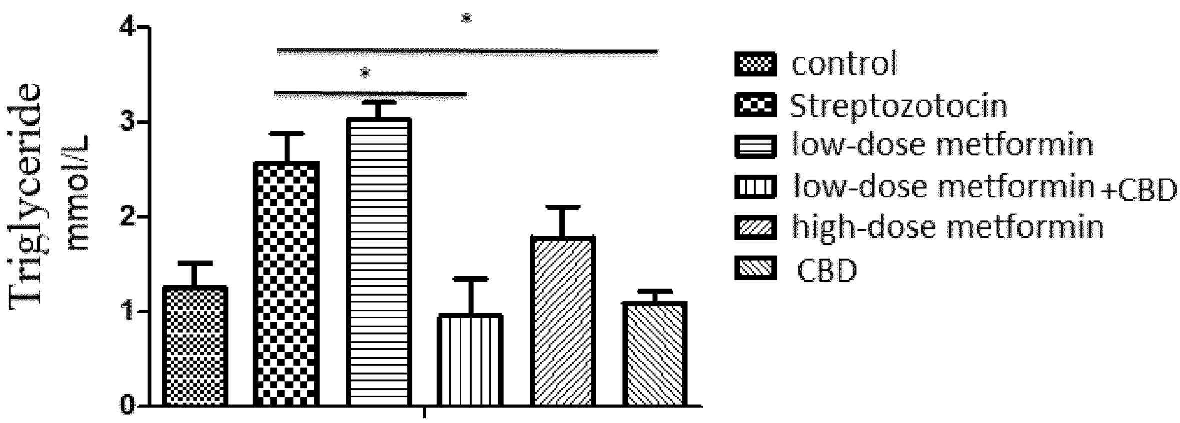


FIG. 6B

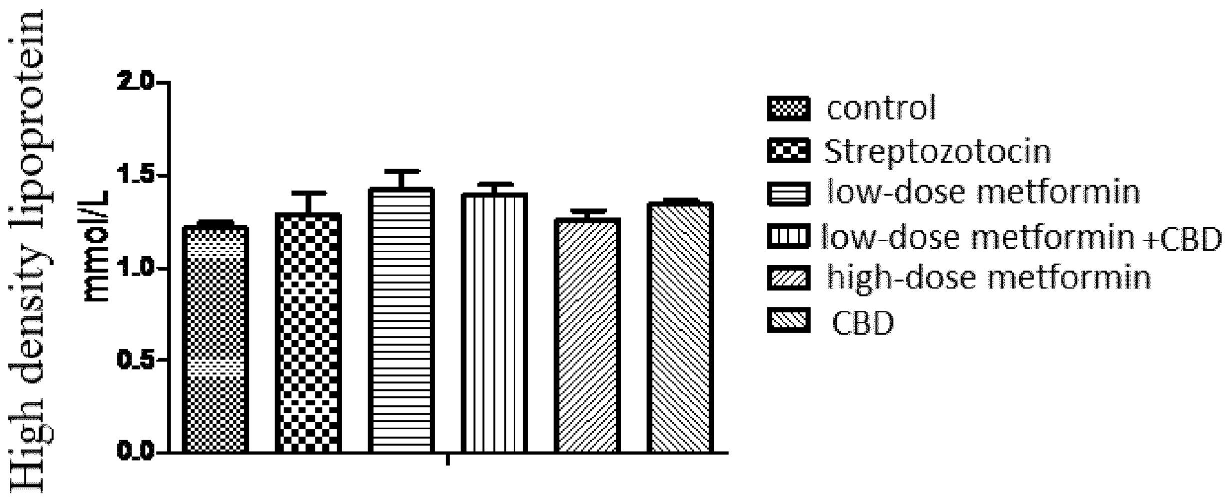


FIG. 6C

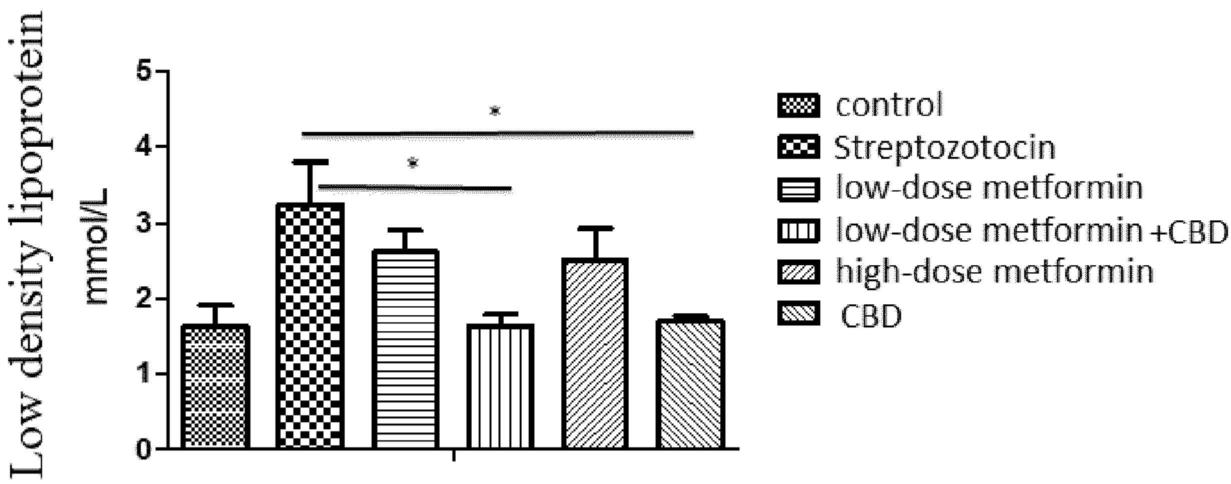


FIG. 6D

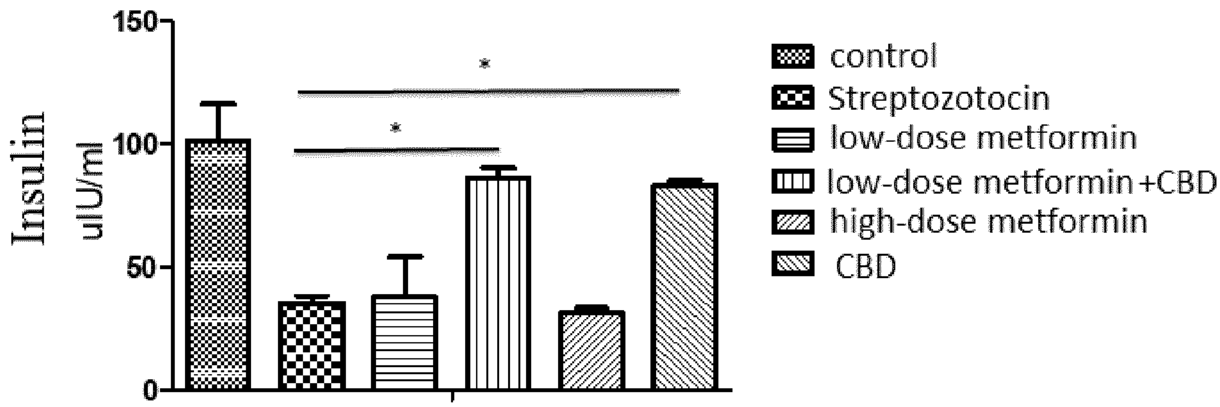


FIG. 6E

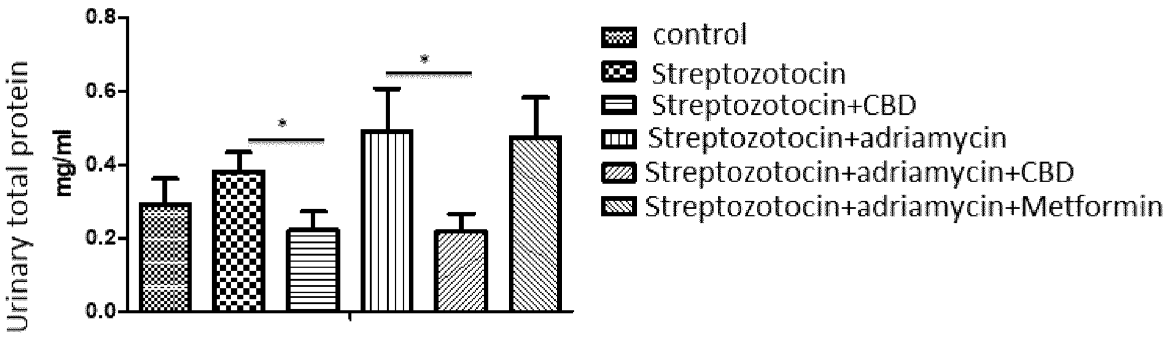


FIG. 7

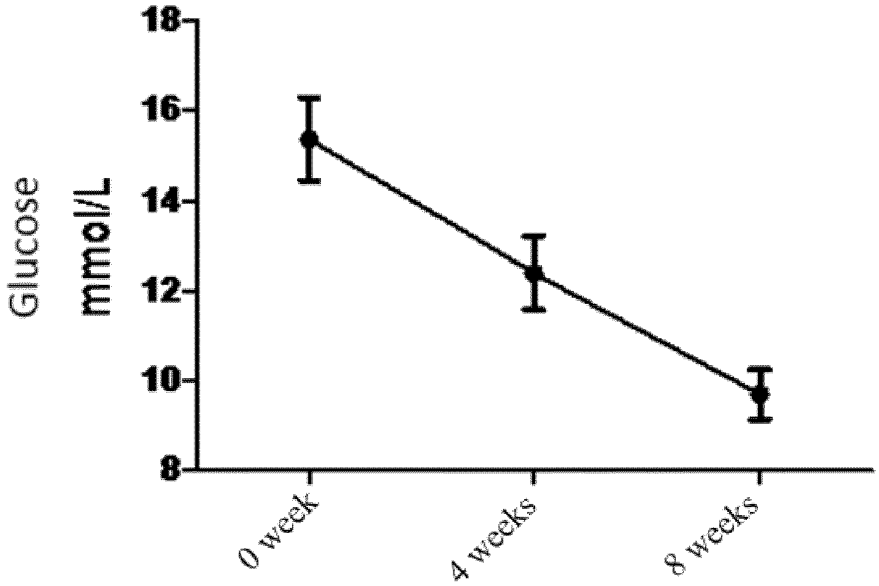


FIG. 8A

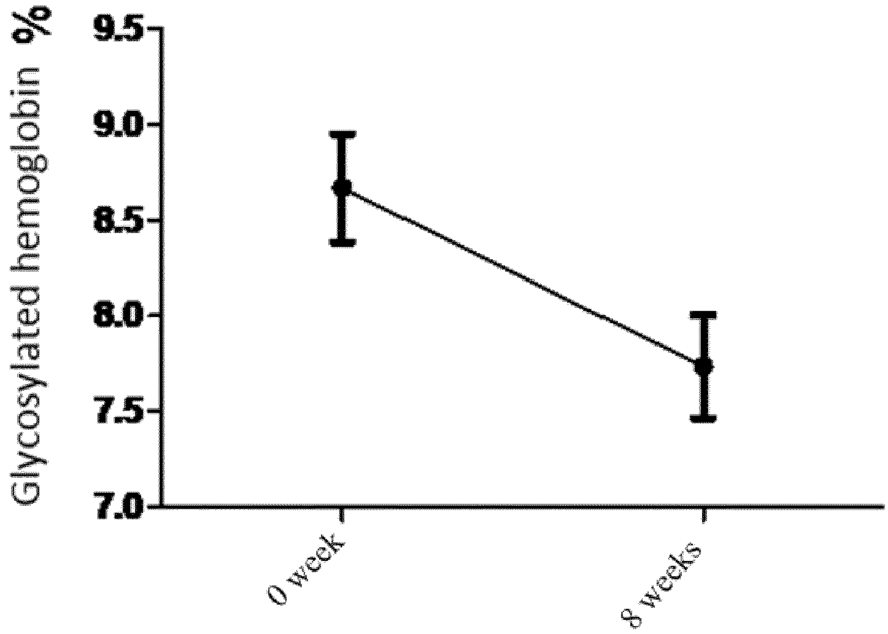


FIG. 8B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018064098 A1